# EUROPÄISCHE PATENTSCHRIFT

(11) **EP 3 613 346 B1**
(45) Veröffentlichungstag und Bekanntmachung des Hinweises auf die Patenterteilung: **16.04.2025**
(21) Anmeldenummer: 18190255.2
(22) Anmeldetag: 22.08.2018
(51) Int. Cl.: A61B 6/04, A61B 6/00, A61B 8/08, A61B 8/00

(54) **VORRICHTUNG UND VERFAHREN FÜR EINE MAMMOGRAPHIEANLAGE**
DEVICE AND METHOD FOR A MAMMOGRAPHY SYSTEM
DISPOSITIF ET PROCÉDÉ POUR UNE INSTALLATION DE MAMMOGRAPHIE

(43) Veröffentlichungstag der Anmeldung: 26.02.2020
(73) Patentinhaber: Siemens Healthineers AG, 91301 Forchheim (DE)
(72) Erfinder: MERTELMEIER, Thomas, 91058 Erlangen (DE); RADICKE, Marcus, 90587 Veitsbronn (DE)
(74) Vertreter: Siemens Healthineers Patent Attorneys

(56) Entgegenhaltungen:
- DE-A1- 102015 218 607
- US-A1- 2005 207 528
- US-A1- 2013 028 373
- US-A1- 2013 129 039
- US-A1- 2017 367 671

## Beschreibung

Die Erfindung betrifft eine Ausgestaltung sowie ein dazugehöriges Verfahren für eine Mammographieanlage.

Zur Lokalisierung und Verifizierung von pathologischen Symptomen bedarf es oftmals unterschiedlicher bildgebender Verfahren um diagnosefähige Bilder von einer Körperregion eines Patienten anzulegen. Für eine erste Diagnose könnten beispielsweise Ultraschallaufnahmen sowie einzelne oder 3D-Röntgenaufnahmen erstellt und unabhängig voneinander auf Bildschirmen wiedergegeben werden. Eine mögliche Zuordnung von Auffälligkeiten in dem Röntgen- bzw. dem Ultraschallbild liegt im Beurteilungsvermögen des behandelnden Arztes. Um eine verifizierte Diagnose beispielsweise nach einer Brustuntersuchung zu stellen, werden neben Mammographie- und/oder Tomosyntheseröntgenaufnahmen auch Ultraschallaufnahmen während einer Sitzung von der Brust eines Patienten angelegt. Für jeden Aufnahmetyp bedarf es jeweils gezielter Vorbereitungen bzw. Voreinstellungen. Sowohl die mit der Röntgentechnologie erstellten Röntgenbilder als auch die mit der Ultraschalltechnologie erstellten Ultraschallbilder können während einer Brustuntersuchung erstellt und einzeln oder gemeinsam bzw. überlagert auf einem Bildschirm zur Beurteilung wiedergegeben werden. Es herrscht in der Fachwelt weitgehende Übereinkunft, dass gegenwärtig nur durch die geschickte Kombination unterschiedlicher Bildgebungsverfahren eine Diagnose mit gleichzeitig hoher Sensitivität und Spezifität erhalten werden kann. Eine Überlagerung von unterschiedlichen Aufnahmetypen setzt eine Fixierung beziehungsweise Komprimierung der Brust beispielsweise zwischen zwei Kompressionsflächen voraus, so dass diese Aufnahmen unmittelbar hintereinander anlegbar sind und das 2D oder 3D-Röntgenbild sowie die Ultraschalldarstellung räumlich zueinander registriert werden können. Bei den entstehenden Aufnahmen aus der Röntgen- und Ultraschalluntersuchung können jeweils sowohl zweidimensionale als auch dreidimensionale Darstellungen entstehen, die dann in bekannter Weise kombiniert oder zumindest räumlich korrespondierend dargestellt werden, um einem Arzt ein Hilfsmittel für eine schnelle und aussagekräftige Diagnose zur Verfügung zu stellen. Während bei Mammographie- bzw. Röntgenbilder für eine Tomosyntheseaufnahme unter anderem eine patientenindividuelle Einstellung wie die Röntgendosis vorgenommen werden muss, muss beim Anlegen einer Ultraschallaufnahme auf eine unmittelbare Übertragung der Ultraschallwellen in und aus dem Brustgewebe geachtet werden. Die Einhaltung dieser Randbedingungen, insbesondere zum Anlegen einer Ultraschallaufnahme, stellt dann hohe Anforderungen an die einzelne Vorrichtung, wenn die Röntgenaufnahme oder Röntgenaufnahmen und die Ultraschallaufnahme unter Beibehaltung der Brustkompression und Brustgeometrie angelegt werden soll. Um eine Gleichheit der Aufnahmegeometrie oder Aufnahmesituation unter der sowohl die Röntgen- als auch die Ultraschalluntersuchung angelegt werden zu gewährleisten, wird der zur Ultraschallabtastung verwendete Schallkopf in die Kompressionseinheit einer Röntgen-Mammographieanlage integriert. Die bisherigen Ausgestaltungen von Ultraschallvorrichtungen bringen jedoch den Nachteil mit sich, dass Randbereiche des Brustgewebes sehr schwer von den Ultraschallwellen erreichbar sind.

Standardmäßig wird zur Reduzierung von Übergangswiderständen für Ultraschallwellen ohne eine Beibehaltung der Brustkompression, ein flüssiges Gel zwischen dem Ultraschallkopf und der Oberfläche der Brust des Patienten aufgebracht und nach der Ultraschallaufnahme wieder entfernt. Diese Vorgehensweise bringt jedoch den Nachteil mit sich, dass eine Registrierung einer Röntgenaufnahme mit einer Ultraschallaufnahme nicht möglich ist.

Eine Vorrichtung für eine Röntgenaufnahme und Ultraschallabtastung einer Brust ist beispielsweise aus der Offenlegungsschrift DE 102012212135 A1 bekannt. Dort ist eine Mammographieanlage mit einem oberen und unteren Kompressionselement ausgebildeten Kompressionssystem, dass sowohl ein Röntgen- und ein Ultraschallsystem aufweist abgebildet und beschrieben. Für die Ultraschallabtastung wird ein an einer Verschiebemechanik befestigter Ultraschallkopf über das obere Kompressionselement bewegt, um dann lotrecht unter dem Schallkopf liegende Bildinformationen aufzunehmen und mit den korrespondierenden Röntgenbildinformationen kombiniert zu werden. Diese Ausgestaltung bringt jedoch bei einer Ultraschallabtastung den Nachteil mit sich, das die im Randbereich zu erfassenden Bildinformationen aufgrund höherer Übergangswiderstände sich verschlechtern können.

Um die Ultraschallwellen vom Ultraschallkopf der Ultraschalleinheit besser in das Gewebe der Brust des Patienten überzuleiten wird wie oben bereits aufgeführt ein Koppelgel verwendet. Das Koppelgel bildet einen geringeren Übergangswiderstand für die vom Ultraschallkopf ausgehenden und von diesem wieder empfangenen Ultraschallwellen. Dieses Koppelgel wird beispielsweise auf die Gaze des oberen Kompressionselementes aufgetragen und diffundiert beispielsweise durch die Gaze in die Übergangsbereiche in denen sich die Brustoberfläche von der Unterseite des oberen Kompressionselementes löst und sich zum unteren Kompressionselement erstreckt. Bei bisher bekannten Kompressionsvorrichtungen wird die Brust komprimiert und damit fixiert, so dass eine Überlagerung des Ultraschalbildes mit dem Röntgenbild möglich ist.

Eine Verbesserung zur Erfassung der Übergangsbereiche wird beispielsweise in der EP Anmeldung 17193206 beschrieben. Dort wird eine Vorrichtung und ein Verfahren mit mindestens einem Luftkissen vorgeschlagen, welches insbesondere Übergangsbereiche der Brust des Patienten an die Unterseite einer mit einer Gaze bespannten oberen Kompressionsvorrichtung drückt um somit eine verbesserte Ankopplung der Ultraschallwellen zu ermöglichen. Bei dieser Vorrichtung wird der Luftdruck in dem oder den Luftkissen standardmäßig angehoben. Trotz der verbesserten Ankopplung können partielle Randbereiche nur eingeschränkt mit der Ultraschallbildgebung erfasst werden.

US20050207528A1 offenbart ein Mammographiegerät mit einem Kompressionselement, welches ein Kissen als Unterkammer aufweist. Das Kissen kann zum Fixieren der Brust aufgeblasen werden. Dabei sind Messgeräte und/oder eine Display-Einheit vorhanden, mit welchen die Kompressionskraft messbar oder steuerbar ist.

US20130129039A1 offenbart ein Mammographiegerät mit einem Kompressionselement, welches ein Kissen unterhalb der oberen Kompressionsplatte aufweist. Das Kissen ist aufblasbar. Der Aufblasvorgang kann manuell oder automatisch erfolgen und anhand der Kompressionskraft oder der Relativposition gesteuert werden.

DE 10 2015 218607 A1 offenbart, dass in der mit einem flexiblen Kompressionselement ausgebildeten Kompressionsmulde einer Kompressionseinheit an der Innenseite der Seitenwände der Kompressionsmulde Sensoren zur Überwachung des flexiblen Kompressionselementes vorgesehen sind.

US 2013/028373 A1 offenbart eine Mammographievorrichtung zum Nachweis von bösartigen Zellen in einer Brust, umfassend eine Röntgenquelle und einen Röntgendetektor, der mit der Röntgenquelle zusammenarbeitet, um ein Röntgenbild der Brust zu liefern, und ferner einen Paddel zum Abflachen durch Drücken der Brust gegen den Röntgendetektor, wobei mindestens ein Sensor zum Messen eines Parameters verwendet wird, der zur Bestimmung des Drucks verwendet wird, bei dem der Paddel die Brust zusammendrückt, und wobei ein Steuerungssystem vorgesehen ist, das die Betätigung des Paddels in Abhängigkeit von dem Druck steuert, der auf die Brust ausgeübt wird, wobei es eine Kontaktflächenmesseinheit zum Messen der Kontaktfläche zwischen der Brust und dem Paddel gibt.

US 2017/367671 A1 offenbart ein Mammographiegerät umfassend: eine bewegliche Einheit, die eine Kompressionsplatte in eine Kompressionsrichtung bewegt, in der die Brust komprimiert wird, und eine Dekompressionsrichtung, in der die Brust dekomprimiert wird; eine Strahlungsquelle; ein Drucksensor, der zur Messung eines Druckdrucks verwendet wird, der eine Drucckraft der Druckplatte pro Flächeneinheit ist; und eine Steuereinheit, die die bewegliche Einheit so steuert, dass die Kompressionsplatte in eine erste Position bewegt wird, in der der vom Drucksensor gemessene Kompressionsdruck ein erster Kompressionsdruck in Kompressionsrichtung ist, und in eine zweite Position bewegt wird, in der der vom Drucksensor gemessene Kompressionsdruck ein zweiter Kompressionsdruck in Dekompressionsrichtung ist, und die eine Steuerung so durchführt, dass Strahlung von der Strahlungsquelle emittiert wird in einem Zustand, in dem sich die Kompressionsplatte an der zweiten Position befindet.

Der Erfindung liegt die Aufgabe zugrunde, eine Vorrichtung und ein Verfahren für eine Ultraschallbildaufnahme derart auszubilden, dass mit einem Ultraschallbild auch die Randbereiche einer Brust eines Patienten wie in einem Röntgenbild erfassbar sind.

Die Aufgabe wird durch die Merkmale des Patentanspruchs 1 oder 8 gelöst.

Mit der dazu vorgesehenen Vorrichtung und dem dazugehörigen Verfahren ist in einer mit mindestens einem Kompressionselement und mit einer mindestens eine Unterkammer aufweisende Positionierungseinheit ausgebildeten Kompressionseinheit zur Komprimierung einer Brust eines Patienten, eine erste Drucksteuerungseinheit vorgesehen, wobei der Druck auf die zu komprimierende Brust des Patienten in der Kompressionseinheit mit einer Aufzeichnungseinheit patientenindividuell überwacht wird. In der ersten Drucksteuerungseinheit sind eine erste und zweite Segmentierungs-/Flächenbestimmeinheit zur Bestimmung einer ersten Fläche und zweiten Fläche integriert. Die erste Fläche wird dabei durch eine Außenkontur der Brust des Patienten sowie die zum Patenten weisende Kante des Kompressionselementes bestimmt und eine zweite Fläche wird durch eine Ist-Kontaktlinie sowie die zum Patenten weisende Kante des Kompressionselementes bestimmt. Eine in der ersten Drucksteuerungseinheit integrierten zweiten Drucksteuerungseinheit verändert den Druck in mindestens einem Luftkissen der Positionierungseinheit derart, dass die darin eingebettete Brust des Patienten an zumindest einem Kompressionselement der Kompressionseinheit gedrückt wird, wobei in mindestens einem ersten Bereich der Druck auf die in der Kompressionseinheit fixierten Brust derart veränderbar ist, dass die Ausmaße der zweiten Fläche an die der ersten Fläche in zumindest diesem ersten Bereich sich annähert.

Erfindungsgemäß ist die mindestens eine Unterkammer während einer Fixierungs- bzw. Komprimierungsphase der Brust mit einem Medium patientenindividuell füllbar. Erfindungsgemäß ist die Abdruckfläche einer zu komprimierenden Brust an das Kompressionselement überwacht und aus den Überwachungsdaten der Anpressdruck der mindestens einen Unterkammer für die Brust des Patienten steuerbar. Erfindungsgemäß überwacht der zumindest eine Sensor die zu komprimierende Brust des Patienten optisch, und die Kompressionseinheit weist zumindest ein mit einer Gaze bespannten Rahmen ausgebildetes Kompressionselement auf, wobei die Gaze derart transparent ausgebildet ist, dass eine Außenkontur der Brust des Patienten sowie eine Ist-Kontaktlinie an der Gaze der zu komprimierenden Brust des Patienten optisch erfasst werden und dass die damit eingeschlossenen Flächen ermittelt werden.

Die Erfindung bringt den Vorteil mit sich, dass die Erfassung der Randbereiche durch die Ultraschallbildgebung dem durch die Röntgenbildgebung erfassbaren Umfang der Brust des Patienten entspricht.

Die Erfindung bringt den Vorteil mit sich, dass an zumindest einer Kompressionsplatte die Abdruckfläche einer zu komprimierenden Brust überwacht und aus den Überwachungsdaten der Anpressdruck für die Brust des Patienten patientenindividuell steuerbar ist.

Die Erfindung bringt den Vorteil mit sich, dass eine Anpressung der Brust des Patienten zumindest an das obere Kompressionselement der Kompressionsvorrichtung optisch überwacht wird.

Die Erfindung bringt den Vorteil mit sich, dass ein steuerbarer Druck patientenindividuell in den beispielsweise aus mehreren Unterkammern gebildeten Positionierungseinheit erhöht, das Volumen von zumindest einer Unterkammer erhöht und dadurch zumindest ein Randbereich der Brust vollständiger an das obere Kompressionselement gedrückt und dieser Bereich auch durch die Ultraschallaufnahme erfassbar ist.

Die Erfindung bringt den Vorteil mit sich, dass während einer Brustuntersuchung die Röntgen- und Ultraschallaufnahme bei einer Fixierungsphase der Brust in einer Kompressionseinheit anlegbar sind und damit die Vorteile der Röntgen- und der Ultraschallaufnahme in einer Darstellung auf einem Bildschirm abbildbar und vom Arzt oder dessen Assistenten für eine Diagnose verwendbar sind.

Die Erfindung bringt den Vorteil mit sich, dass ein steuerbarer Druck in der Kammer oder den Unterkammern der Positionierungseinheit patientenindividuell aufbaubar ist.

Die Erfindung bringt den Vorteil mit sich, dass der auf das Gewebe der Brust eines Patienten ausübbare Druck patientenindividuell und partiell steuerbar ist.

Die Erfindung bringt den Vorteil mit sich, dass die zur Drucksteuerung in einer Kammer oder den Unterkammern der Positionierungseinheit erforderlichen Daten mittels optischer Überwachung der Anpressfläche des Gewebes der Brust an einem Kompressionselement abgeleitet oder ermittelbar ist.

Die Erfindung bringt den Vorteil mit sich, dass die zur Druckanpassung erforderlichen Daten mit in bzw. auf oder an den Kompressionsplatten integrierter Sensoren ermittelbar sind.

Vorteilhafte Ausgestaltungen der erfindungsgemäßen Vorrichtung und des dazugehörigen Verfahrens sind jeweils Gegenstand von weiteren Patentansprüchen.

Nachfolgend wird ein Ausführungsbeispiel der Erfindung anhand von Zeichnungen, ohne jedoch darauf beschränkt zu sein, näher erläutert.

Es zeigen:
Figur 1 eine Mammographieanlage,
Figur 2 eine Draufsicht,
Figur 3 eine weitere Draufsicht,
Figur 4 eine Seitenansicht und
Figur 5 ein Blockdiagramm.

Bei dieser Vorrichtung und dem dazugehörigen Verfahren wird durch Auswertung von Videoaufnahmen von einer Kompression einer Brust eines Patienten in einer Kompressionseinheit eine Ist-Kontaktfläche des an eine Gaze eines Kompressionselementes gedrückten Gewebes der Brust des Patienten eine Volumen- und damit eine Druckveränderung in mindestens einer Unterkammer einer in der Kompressionseinheit integrierten Positionierungseinheit derart herbeigeführt, dass die Ist-Kontaktfläche einer an der Außenkontur der Brust orientierten maximal möglichen ersten Fläche entspricht.

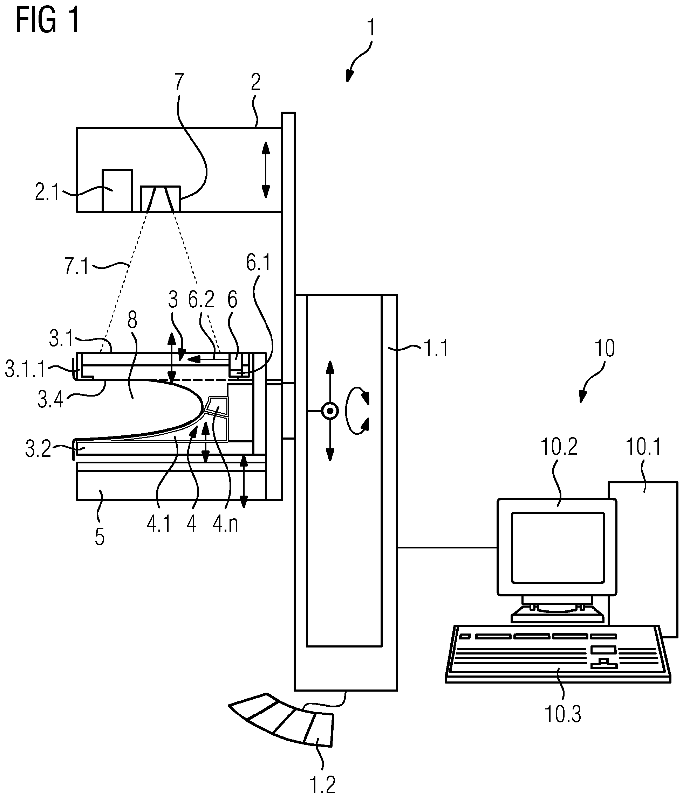

In der Figur 1 ist eine Mammographieanlage 1 schematisch abgebildet. In dieser Mammographieanlage 1 sind eine aus Röntgenquelle 2.1 und einem Detektor 5 gebildete Röntgeneinheit sowie eine in eine aus einem oberen und unteren Kompressionselement 3.1, 3.2 gebildeten Kompressionseinheit 3 integrierten Ultraschalleinheit 6 angeordnet. Gesteuert wird die Mammographieanlage 1 mittels Steuerungsprogramme die beispielsweise im Stativ der Mammographieanlage 1 selbst und/oder in einer mit der Mammographieanlage 1 verbundenen Datenverarbeitungseinheit 10 ablaufen. Auf einer Visualisierungseinheit 10.2 der Datenverarbeitungseinheit 10 können die in einer Prozessoreinheit 10.1 der Datenverarbeitungseinheit 10 mit Bildverarbeitungsprogrammen bearbeiteten Mammographie- und/oder Tomosyntheseröntgenbilder, die Ultraschallbilder oder Videobilder einzeln, kombiniert und/oder überlagert von einem Betrachter ausgewählt und angezeigt werden. In der Datenverarbeitungseinheit 10 sind die Videobilder einer nahe der Röntgenquelle 2.1 integrierten Aufzeichnungseinheit 7 in Echtzeit bearbeitbar. Die Kompressionseinheit 3 der Mammographieanlage 1 kann beispielsweise über Eingabemittel 10.3 an der Datenverarbeitungseinheit 10 und/oder über Funktionsschalter unmittelbar an der Mammographieanlage 1 und/oder zumindest einem Fußschalter 1.2 gesteuert bzw. bedient werden. Für eine Röntgen- und/oder Ultraschallaufnahme wird die Brust eines Patienten 8 beispielsweise in der Kompressionseinheit 3 fixiert. Fixiert bzw. komprimiert wird dabei das Gewebe der Brust des Patienten 8 beispielsweise zwischen einem mit einer Gaze 3.4 bespannten Rahmen 3.1.1 eines oberen Kompressionselementes 3.1 und einem unteren Kompressionselement 3.2. Als unteres Kompressionselement 3.2 kann auch die Oberfläche eines Detektors 5 verwendet werden. Der mit der Gaze 3.4 bespannte Rahmen 3.1.1 des oberen Kompressionselementes 3.1 kann auch als obere Kompressionsplatte bezeichnet werden. Um die Ultraschallwellen des Ultraschallkopfes 6.1 der Ultraschalleinheit 6 besser dem Gewebe der Brust des Patienten 8 zuzuleiten wird beispielsweise ein Koppelgel auf die Gaze 3.4 und/oder auf das Brustgewebe der Brust des Patienten 8 aufgetragen. Bewegt werden kann die Ultraschalleinheit 6 in den angegebenen Richtungen 6.2. Das Koppelgel wird nach einer Röntgenaufnahme und vor einer Ultraschallaufnahme auf die Gaze 3.4 aufgetragen und diffundiert beispielsweise teilweise durch diese auf die Gewebeoberfläche der Brust des Patienten 8 und bildet so ein Koppelmedium zwischen der Ultraschalleinheit 6 und dem Brustgewebe wobei die Übergangswiderstände für die vom Ultraschallkopf 6.1 ausgehenden und von diesem wieder empfangenen Ultraschallwellen verringert werden. Zwischen dem oberen und unteren Kompressionselement 3.1, 3.2 ist eine aus mindestens einer Unterkammer 4.1, ..., 4.n gebildetes Positionierungseinheit 4 angeordnet. Die mindestens eine Unterkammer 4.1, ..., 4.n der Positionierungseinheit 4 wird mit einem Medium während der Fixierungs- bzw. Komprimierungsphase patientenindividuell gefüllt. Im Röntgengehäuse 2 ist beispielsweise nahe dem Röntgenkopf 2.1 eine den Kompressionsvorgang aufzeichnende Aufzeichnungseinheit 7 integriert. Der Öffnungswinkel der Aufzeichnungseinheit 7 ist derart gewählt, dass der Aufzeichnungs- bzw. Aufnahmebereich 7.1 zumindest die Rückseite des oberen Kompressionselementes 3.1. erfasst. Das obere Kompressionselement 3.1 ermöglicht, da bestehend aus einer in einem Rahmen 3.1.1 eingespannten Gaze 3.4, das Erkennen der in bzw. auf der auf dem unteren Kompressionselement 3.2 in der Positionierungseinheit 4 eingebetteten bzw. aufliegenden Brust des Patienten 8. Wird die Brust für eine Röntgen- und/oder Ultraschallaufnahme fixiert so wird diese in einem ersten Kompressionsschritt zwischen dem unteren und oberen Kompressionselement 3.1, 3.2 komprimiert bzw. fixiert. In einem weiteren Schritt wird der Druck und damit das Volumen in mindestens einer Unterkammer 4.1,**...**,4.n der Positionierungseinheit 4 unter optischer Überwachung patientenindividuell verändert.

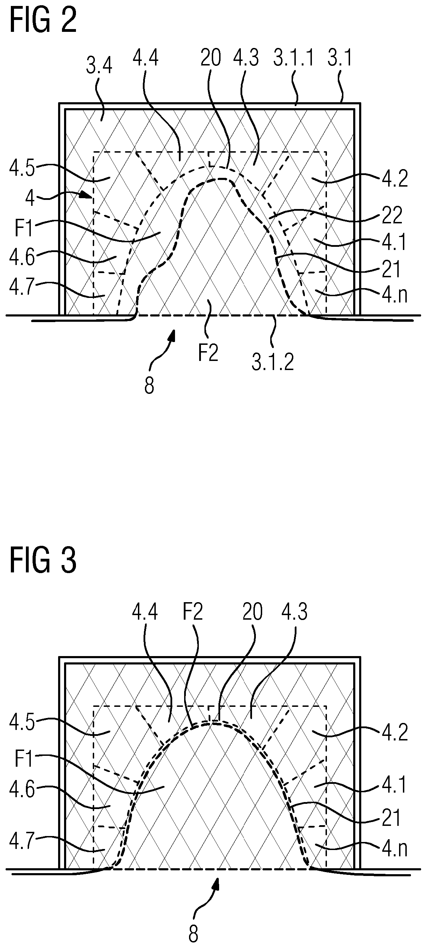

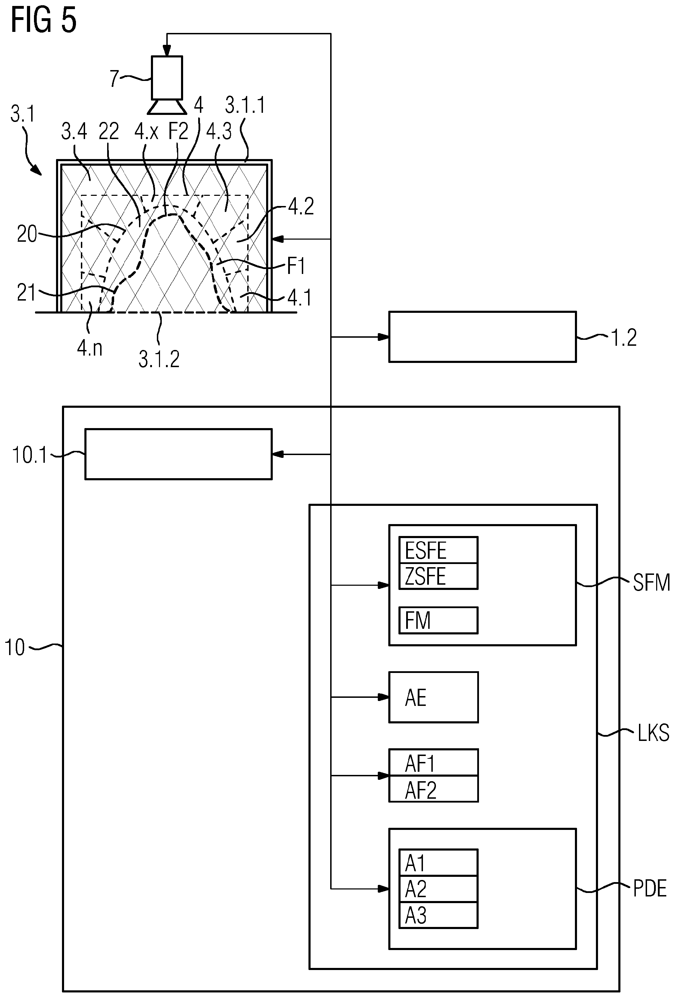

In Figur 2 ist der in Fig. 1 angedeutete Aufzeichnungs- bzw. Aufnahmebereich 7.1 der Aufzeichnungseinheit 7 wiedergegeben. Die Ausmaße des Aufzeichnungsbereichs 7.1 sowie die Auflösung der Aufzeichnungseinheit 7 sind so eingestellt, dass die Kontur der Brust sowie der Abdruck der Brust des Patienten 8 in der im Rahmen des oberen Kompressionselementes 3.1.1 eingespannten Gaze 3.4 optisch erfasst und mit Bildbearbeitungsprogrammen jeweils selektierbar bzw. abbildbar ist. Angedeutet im linken oberen Eck des Rahmens 3.1.1 des oberen Kompressionselementes 3.1 ist die in diesem eingespannte Gaze 3.4. Abgebildet ist auch die Positionierungseinheit 4 mit den angedeuteten einzelnen Unterkammern 4.1, ..., 4.n. Die Unterkammer oder die Unterkammern 4.1,..., 4.n, beispielsweise mit Luft gefüllt, weisen zu Beginn der Kompressionsphase eine flexible Form mit zumindest einen vorgebbaren Druck auf. Die einzelnen Unterkammern 4.1, ..., 4.n der Positionierungseinheit 4 passen sich der jeweiligen Form der Brust des Patienten 8 an und bewirken bei Erhöhung des Drucks in der oder den Unterkammern 4.1,..., 4.n jeweils eine Vergrößerung von diesen und dadurch eine Anhebung und Anpressung des Brustgewebes an beispielsweise das obere Kompressionselement 3.1. Als Medium zur Druckerhöhung kann neben Luft beispielsweis auch temperiertes Wasser oder ein flüssiges Gel verwendet werden. Das Medium in den Unterkammern 4.1,..., 4.n kann unterschiedlichen Druck und dadurch unterschiedliches Volumen aufweisen. Anstatt einer Vielzahl von einzelnen Unterkammern 4.1,...,4.n kann eine Untermenge oder eine einzelne Unterkammer als Positionierungseinheit 4 auf dem unteren Kompressionselement 3.2 angeordnet sein. Eine Verringerung des Abstandes zwischen dem oberen und unteren Kompressionselement 3.1, 3.2 oder einer Vergrößerung der Positionierungseinheit 4 führt zu einer Fixierung und damit zu einer Komprimierung der Brust des Patienten 8. Das Gewebe der Brust wird dadurch gegen das obere Kompressionselement 3.1 und in die auf dem unteren Kompressionselement 3.2 angeordneten Positionierungseinheit 4 gedrückt. In einer Abstandsermittlungseinheit AE wird die aktuelle Brustdicke durch eine Abstandsmessung zwischen dem ersten und zweiten Kompressionselement 3.1, 3.2 ermittelt. Durch eine weitere Verringerung des Abstandes wird die Brust des Patienten mehr in die formgebende Positionierungseinheit 4 gedrückt. Ein durch das Drücken der Brust des Patienten 8 an die Gaze 3.4 im oberen Kompressionselement 3.1 sich bildender Abdruck bildet eine Ist-Kontaktline 21. Diese Ist-Konturlinie 21 sowie die zum Patienten weisende Kante 3.1.2 des Rahmens des oberen Kompressionselementes 3.1 schließen eine als zweite Fläche F2 bezeichnete Ist-Kontaktfläche ein. Weiter ist in dieser Figur die mit der Aufzeichnungseinheit 7 selektierbare Außenkontur 20 der Brust des Patienten 8 angedeutet. Diese Außenkontur 20 der Brust des Patienten 8 kann ebenso als der äußerste Brustbereich der keinen direkten Kontakt zur Gaze 3.4 des oberen Kompressionselementes 3.1 hat umschrieben werden. Erkennbar und mittels entsprechender Bildverarbeitungsprogramme kann die Außenkontur 20 sowie die Ist-Kontaktlinie 21 auf einer Visualisierungseinheit 10.2 wiedergegeben werden. Zwischen der Außenkontur 20 der Brust des Patienten 8 und der Ist-Konturlinie 21 der Brust an der Gaze 3.4 befindet sich der Brustbereich 22 der keinen direkten Kontakt zur Gaze 3.4 des oberen Kompressionselementes 3.1 hat. Dieser Bereich 22 soll minimiert werden. Mit einem eine erste Segmentierungs-/Flächenbestimmeinheit ESFE sowie eine zweite Segmentierungs-/Flächenbestimmeinheit ZSFE aufweisenden Segmentierungs-/Flächenermittlungsmodul SFM, siehe Fig. 5, werden durch die Außenkontur 20 und die zum Patienten weisende Kante des oberen Kompressionselementes 3.1.2 eingeschlossene erste Fläche F1 und die durch die Ist-Konturlinie 21 und die zum Patienten weisende Kante des oberen Kompressionselementes 3.1.2 eingeschlossene zweite Fläche F2 ermittelt. Die erste Fläche F1 gibt dabei die maximal möglich abbildbare Brustfläche an. Die zweite Fläche F2 kann auch als Ist-Kontaktfläche des Brustgewebes mit der im Rahmen eingespannten Gaze 3.4 bezeichnet werden. Die Ist-Kontaktlinie 21 ist in dieser Darstellung mit einer deutlichen Abweichung zur Außenkontur 20 der Brust des Patienten 8 dargestellt. Abgebildet ist auch die Positionierungseinheit 4 mit den einzelnen Unterkammern 4.1, ...., 4.n. Eine Ausführungsvariante mit einer Positionierungseinheit 4 mit einer einzigen Kammer ist ebenso möglich. Während der Kompression wird beispielsweise nach einem Erreichen eines der Brust des Patienten 8 angepassten Mindestabstandes zwischen dem oberen und unteren Kompressionselement 3.1, 3.2, in ausgewählten Bereichen steuernd eine Volumenerweiterung vorgenommen währenddessen kontinuierlich die erste und zweie Fläche F1, F2 errechnet sowie in einem Flächenvergleichsmodul FM der Quotient aus zweiter F2 zur ersten F1 bestimmt wird. Dessen Wert wird in Abhängigkeit zur Kompressionskraft/Druck in der oder den Unterkammern der Positionierungseinheit 4 sowie der Brustdicke berechnet bzw. dargestellt. Falls bei steigendem Druck in den Unterkammern 4.1,...,4.n der Positionierungseinheit 4 der Quotient F2/F1 unverändert bleibt, wird vom Flächenvergleichsmodul FM bei Überschreitung eines vorgebaren Schwellwertes eine weitere Druckerhöhung in den Unterkammern 4.1,..., 4.n gestoppt. Die Fixierung bzw. Komprimierung der Brust des Patienten erfolgt mittels einer Mikroprozessorsteuerung. Mikroprozessorgesteuert erfolgt auch die bereits oben angeführte Druck- bzw. Volumenvergrößerung in der oder den Unterkammern 4.1,...,4.n der Positionierungseinheit 4. Während mit einer ersten Drucksteuereinheit LKS Videoaufnahmen während einer Abstandsverringerung zwischen dem oberen und unteren Kompressionselement 3.1, 3.2 ausgewertet die Abstandsverringerung gestoppt wird übernimmt eine zweite Drucksteuereinheit PDE, siehe Fig. 5, die oben aufgeführt patientenindividuelle Anpressung der Brust des Patienten 8 an das obere Kompressionselement 3.1. wobei der Brustbereich 22 der keinen direkten Kontakt zur Gaze 3.4 des oberen Kompressionselementes 3.1 hat verringert wird. Die patientenindividuelle Druck- bzw. Volumenveränderung in der oder den Unterkammern 4.1,..., 4.n der Positionierungseinheit 4 erfolgt auf der Grundlage einer kontinuierlichen Videobildauswertung. Durch die kontinuierliche Bildauswertung werden die Abstände zwischen Außenkontur 20 und Ist-Konturline 21 ermittelt und eine Annäherung der Ist-Konturline 21 an die Außenkontur 20 mit der zweiten Drucksteuerungseinheit PDE in einem ersten, zweiten und dritten Abschnitt durchgeführt. In einer weiteren Ausführungsvariante wird der oder die Bereiche 22 der Brust des Patienten 8 der noch keinen direkten Kontakt zur Gaze 3.4 des oberen Kompressionselementes 3.1 hat oder haben ermittelt. Im Falle, dass die Positionierungseinheit 4 aus mehreren Unterkammern 4.1,...,4.n besteht, wählt die zweite Drucksteuerungseinheit PDE in Verbindung mit der ersten Segmentierungs-/Flächenbestimmeinheit ESFE und/oder die zweite Segmentierungs-/Flächenbestimmeinheit ZSFE die Unterkammer 4.x oder die Unterkammern 4.x,...,4.y aus in dem oder denen die Bereiche 22 der Brust des Patienten 8 noch keinen direkten Kontakt zur Gaze 3.4 des oberen Kompressionselementes 3.1 haben. Die Druck- bzw. die Volumenvergrößerung in den einzelnen Unterkammern 4.x erfolgt beispielsweise durch Überwachung des oder der Abstände zwischen der Außenkontur 20 und der Ist-Kontaktlinie 21. In Bereichen in denen die Abweichungen am Größten sind oder ein vorgebbarer Abstand überschritten wird, wird oder werden die unmittelbar darunter liegende sowie die angrenzenden Unterkammern der Positionierungseinheit 4 ermittelt und der Druck- bzw. das Volumen in den betroffenen Unterkammern erhöht. Je nach Abstand des oberen zum unteren Kompressionselementes 3.1, 3.2 kann eine Kompressionsbedingung für einen ersten, zweiten oder dritten Abschnitt erfüllt sein. Die zweite Drucksteuerungseinheit PDE fährt dann abhängig von den Auswertungen der Videoaufzeichnungen der Aufzeichnungseinheit 7 mit dem noch fehlenden Abschnitt oder Abschnitten fort. Ausgehend von einem erreichten von der Brust des Patienten 8 abhängigen Mindestabstandes zwischen dem oberen und unteren Kompressionselement 3.1, 3.2 beginnt die patientenindividuelle Kompressionsoptimierung mit einem der Abschnitte. Im ersten Abschnitt A1 wird in den Unterkammern 4.1,..., 4.n der Druck kontinuierlich solange erhöht, solange die Ist-Kontaktfläche F2 kleiner 80% der ersten Fläche F1 ist. In dem zweiten Abschnitt A2 erfolgt die Druckerhöhung mit reduzierter Geschwindigkeit und/oder partiell in den Unterkammern bzw. der Unterkammer, sobald die Ist-Kontaktfläche F2 größer/gleich 80% und kleiner 95% der ersten Fläche F1 beträgt. In dem dritten Abschnitt A3, in dem die Ist-Kontaktfläche F2 größer/gleich 95% bis 100% der ersten Fläche F1 einnimmt erfolgt eine weitere Druckerhöhung in den oder der Unterkammer sehr langsam. Die zweite Drucksteuerungseinheit PDE verhindert einen weiteren Druckanstieg wenn schon alle Brustbereiche Kontakt zur Gaze 3.4 des oberen Kompressionselementes 3.1 haben. Eine entsprechende Signalisierung wird auf der Visualisierungseinheit 10.2 der Datenverarbeitungsanlage 10 an den Bediener der Mammographieanlage 1 abgegeben. Entspricht der Abstand zwischen der Außenkontur der Brust des Patienten 20 und der Ist-Kontaktline 21 den Kriterien, beispielsweise eines vorgebaren Minimums, so wird die Druckerhöhung in den selektierten Unterkammern der Positionierungseinheit 4 gestoppt.

Mechanische oder elektronische Sicherheitsvorkehrungen werden durch Aktivierung von Steuertasten und/oder durch betätigen des Fußschalter 1.2 aktiviert.

In Figur 3 ist die maximal erreichbare Größe der zweiten Fläche F2 erreicht. Die Größe der zweiten Fläche F2 entspricht der Größe der ersten Fläche F1. Die mögliche Flächengleichheit bzw. die Annäherung der zweiten Fläche F2 an die Größe der ersten Fläche F1 wird steuernd durch eine erste/zweite Drucksteuerungseinheit LKS,PDE erwirkt und abgeschlossen. Die Ist-Kontaktlinie 21 der Brust des Patienten 8 entspricht im Idealfall der Außenkontur 20 der Brust des Patienten 8.

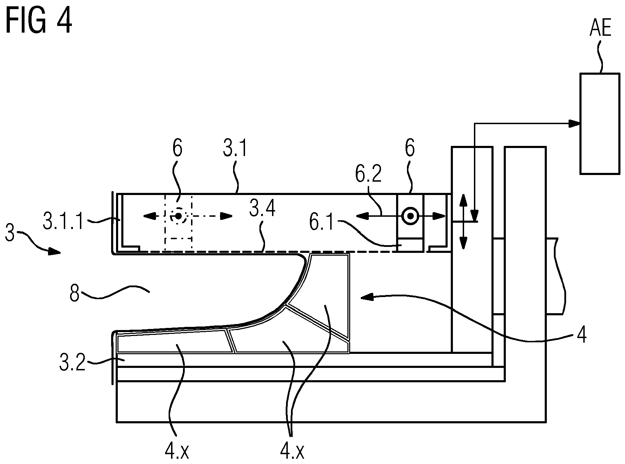

In Figur 4 ist eine Seitenansicht auf die Kompressionseinheit 3 mit einer mehrere Unterkammern 4.x aufweisenden Positionierungseinheit 4 sowie einer Ultraschalleinheit 6 widergegeben. Die Form der Einbettung der Brust des Patienten 8 durch die Positionierungseinheit 4 kann konvex oder segmentweise konvex bzw. linear verlaufen.

In Figur 5 ist eine Steuereinheit sowie weitere Substeuereinheiten, beziehungsweise Steuermodule, für eine patientenabhängige Brustkompression sowie das durch eine Aufzeichnungseinheit 7 erfasste obere Kompressionselement 3.1 der Kompressionseinheit 3 schematisch dargestellt. Als Steuereinheit sind schematisch einer ersten Drucksteuerungseinheit LKS Substeuereinheiten wie Segmentierungs-/Flächenermittlungsmodul SFM sowie eine zweite Drucksteuerungseinheit PDE zugeordnet. Die erste sowie zweite Drucksteuerungseinheit LKS, PDE führen patientenindividuell die Komprimierung der Brust des Patienten 8 durch. Der Komprimierungsvorgang zur Komprimierung der Brust wird dabei so lange weitergeführt bis das Gewebe der Brust des Patienten 8 an zumindest einem Kompressionselement 3.1, 3.2 sich großflächig anfügt, siehe Fig. 3. Ein Abbruch des Kompressionsvorgangs ist zu jedem Zeitpunkt möglich. Die Aufzeichnungseinheit 7 ist derart ausgerichtet, dass der Aufzeichnungs- bzw. Aufnahmebereich 7.1 zumindest die Rückseite der in einen Rahmen 3.1.1 des oberen Kompressionselementes 3.1 eingespannten Gaze 3.4 wiedergibt. Die verwendete Gaze 3.4 ist beispielsweise semitransparent und ermöglicht unter anderem die optische Erfassung des zwischen dem oberen und unteren Kompressionselementes 3.1, 3.2 angeordneten Positionierungselementes 4 sowie die darin eingebettete Brust des Patienten 8 und dessen Umriss bzw. Außenkontur 21 sowie dessen Abdruck an der Gaze 3.4. Eine Verringerung des Abstandes zwischen dem oberen und unteren Kompressionselement 3.1, 3.2 bewirkt zum einen eine Fixierung und zum anderen eine Komprimierung der Brust des Patienten 8. Das Gewebe der Brust des Patienten 8 wird dabei sowohl gegen das obere Kompressionselement 3.1 und in die auf dem unteren Kompressionselement 3.2 aufliegende Positionierungseinheit 4 gedrückt. Das an die Gaze 3.4 gedrückte Gewebe der Brust des Patienten 8 ist in der Figur 5 fett schraffiert dargestellt. Die Fläche des Abdrucks wird mit zweite Fläche F2 bezeichnet. Die zweite Fläche F2 erstreckt sich zwischen der durch die Ist-Kontaktlinie 21 sowie die zum Patienten weisende Kante 3.1.2 des oberen Kompressionselementes 3.1 gebildeten Eingrenzung. Durch die Aufzeichnungseinheit 7 ebenso erfassbar ist die Positionierungseinheit 4 sowie die einzelnen, soweit vorhandenen, Unterkammern 4.1, ..., 4.n. Die einzelnen Unterkammern 4.1, ..., 4.n der Positionierungseinheit 4 sind in dieser Darstellung vorgeformt oder passen sich der jeweiligen Form der Brust des Patienten 8 an und bewirken bei Erhöhung des Drucks eine Volumenvergrößerung der jeweiligen Unterkammer und somit eine Anhebung des Brustgewebes. Das Volumen bzw. der Druck kann beispielsweise durch eine Zuführung von Luft in die Unterkammern 4.1,..., 4.n oder einem Fluid wie Wasser oder einem dünnflüssigen Gel verändert werden. Anstatt einer Vielzahl von einzelnen Unterkammern 4.1,**...**,4.n kann wie bereits angeführt eine Untermenge oder eine einzelne bzw. einstückige Positionierungseinheit 4 auf dem unteren Kompressionselement 3.2 angeordnet sein. Durch die Aufzeichnungseinheit 7 werden jeweils die Außenkontur 20, die Ist-Kontaktlinie 21 sowie die zum Patienten weisende Kante 3.1.2 des oberen Kompressionselementes 3.1 erfasst und die Bilddaten an das Segmentierungs-/Flächenermittlungsmodul SFM weitergeleitet und ausgewertet. Die erste Segmentierungs-/Flächenbestimmeinheit ESFE segmentiert die Außenkontur 20 und die zum Patienten weisende Kante 3.1.2 des oberen Kompressionselementes 3.1 und ermittelt aus der aktuellen Außenkontur 20 eine mit erste Fläche F1 bezeichnete maximal möglichen Flächigkeit der jeweils zu komprimierenden Brust des Patienten 8. Die zweite Segmentierungs-/Flächenbestimmeinheit ZSFE segmentiert die Ist-Kontaktlinie 21 und die zum Patienten weisende Kante 3.1.2 des oberen Kompressionselementes 3.1 und berechnet kontinuierlich die von der Kante 3.1.2 des oberen Kompressionselementes 3.1 und der Ist-Kontaktline 21 eingeschlossene zweite Fläche F2. Die Außenkontur 20 sowie die zum Patienten weisende Kante 3.1.2 ist mit einer unterbrochenen Linie, die Ist-Kontaktlinie 21 mit einer durchgehenden Linie in dem auf der Visualisierungseinheit 10.2 der Datenverarbeitungseinheit 10 wiedergegebenen Videobild der Aufzeichnungseinheit 7 hervorgehoben. Die erste Fläche F1 gibt dabei die maximal mögliche Brustfläche und die zweite Fläche F2 die Fläche des Abdrucks der Brustoberseite an der im Rahmen 3.1.1 des oberen Kompressionselementes 3.1 eingespannten Gaze 3.4 wieder. Während der Kompression der Brust des Patienten 8 wird kontinuierlich die erste und zweite Fläche F1, F2 von der jeweiligen Segmentierungs-/Flächenbestimmeinheit ESFE; ZSFE sowie der Quotient aus der zweiten Fläche F2 zur ersten Fläche F1 in einem Flächenvergleichsmodul FM berechnet. Der Quotient aus der zweiten Fläche zur ersten Fläche F2/F1 wird unter Berücksichtigung der Kompressionskraft bzw. dem Druck in der Positionierungseinheit 4 beziehungsweise der oder den Unterkammern 4.1,... , 4.n der Positionierungseinheit 4 sowie der ermittelten aktuellen Brustdicke berechnet. Die Kompressionskraft könnte beispielsweise auch mittels in der Kompressionseinheit 3 integrierter Drucksensoren erfasst werden. Die aktuell vorliegende Brustdicke wird in einer Abstandsermittlungseinheit AE durch eine Abstandsmessung zwischen dem oberen und unteren Kompressionselement 3.1, 3.2 ermittelt. Falls bei einem sich weiter verringernden Abstand des oberen zum unteren Kompressionselement 3.1, 3.2 keine Vergrößerung der zweiten Fläche F2 erfolgt, wird vom Flächenvergleichsmodul FM bei Überschreitung eines vorgebbaren Schwellwertes eine weitere Druckerhöhung durch eine Abstandsverringerung auf das Gewebe der Brust des Patienten 8 gestoppt. Ein Stopp der Druckerhöhung wird durch eine erste Abschalteeinheit AF1 erwirkt. Die patientenindividuelle Kompression der Brust wird mit der, wie auch zur Figur 2 bereits angedeutet bzw. ausgeführt, mit der in der ersten Drucksteuerungseinheit LKS integrierten zweiten Drucksteuerungseinheit PDE regelnd überwacht und gesteuert.

Mittels einer kontinuierlichen Bild-, Sensor- und/oder Druck- bzw. Messdatenauswertung wie beispielsweise im Segmentierungs-/Flächenermittlungsmodul SFM sowie im Flächenvergleichsmodul FM führt die erste Drucksteuerungseinheit LKS in den Unterkammern 4.1,...,4.n der Positionierungseinheit 4 steuernd eine Druckerhöhung durch Verringerung des Abstandes des oberen und unteren Kompressionselementes 3.1, 3.2 durch. Bei diesem Vorgang wird eine flächenmäßige Annäherung der zweiten Fläche F2 an die erste Fläche F1 erreicht. Eine weiterführende flächenmäßige Annäherung zwischen der zweiten und ersten Fläche F1, F2 wird mit den der zweiten Drucksteuerungseinheit PDE zugeordneten Steuermodulen erreicht. Die zweite Drucksteuerungseinheit PDE kann auch als partielle Drucksteuerungseinheit bezeichnet werden. Nachfolgend ist der von der zweiten Drucksteuerungseinheit PDE durchgeführte Anpassungs- bzw. Optimierungsprozess in einen ersten, zweiten und dritten Abschnitt A1, A2 und A3 unterteilt. Die Ausbildung eines ersten, zweiten und dritten Abschnittes ist beispielhaft. Im ersten Abschnitt A1 wird in den Unterkammern 4.1,..., 4.n, wie bereits aufgeführt, der Druck, beispielsweise durch eine Abstandsverringerung zwischen dem oberen und unteren Kompressionselement 3.1, 3.2 oder einer Volumenvergrößerung der Unterkammern 4.1,..., 4.n, kontinuierlich solange erhöht, solange die Ist-Kontaktfläche F2 kleiner 80% der ersten Fläche F1 ist. In dem zweiten Abschnitt A2 erfolgt die Druckerhöhung mit reduzierter Geschwindigkeit und/oder partiell in den Unterkammern bzw. einer Unterkammer, sobald die Ist-Kontaktfläche F2 größer/gleich 80% und kleiner 95% der ersten Fläche F1 beträgt. In dem dritten Abschnitt A3, erfolgt die Druckerhöhung, beispielsweise durch eine partielle Volumenerweiterung in der oder den Unterkammern 4.1,..., 4.n der Positionierungseinheit 4 sehr langsam, bis die Ist-Kontaktfläche F2 größer/gleich 95% bis 100% der ersten Fläche F1 einnimmt wobei das Volumen in mindestens einem Luftkissen (4.1, ...,4.n) der Positionierungseinheit (4) derart verändert wird, dass die darin eingebettete Brust des Patienten (8) an das zumindest eine Kompressionselement (3.1, 3.2) der Kompressionseinheit (3) gedrückt wird, wobei in mindestens einem ersten Bereich der Druck auf die in der Kompressionseinheit (3) fixierte Brust derart veränderbar ist, dass die Ausmaße der zweiten Fläche (F2) die der ersten Fläche (F1) in zumindest diesem ersten Bereich angenähert wird. Eine großflächige Anfügung der Brust des Patienten liegt dann vor, wenn die zweite Fläche F2 der ersten Fläche F1 entspricht, siehe Fig. 3. Die zweite Drucksteuerungseinheit PDE verändert das Volumen in mindestens einem Luftkissen 4.1,...,4.n der Positionierungseinheit 4 derart, dass die darin eingebettete Brust des Patienten 8 an das zumindest eine Kompressionselement 3.1, 3.2 der Kompressionseinheit 3 gedrückt wird, wobei in mindestens einem selektierten Bereich der Druck auf die in der Kompressionseinheit 3 fixierten Brust derart veränderbar ist, dass die Ausmaße der zweiten Fläche F2 die der ersten Fläche F1 in zumindest diesem selektierten Bereich entsprechen. Die zweite Drucksteuerungseinheit PDE verhindert einen weiteren Druckanstieg wenn eine Flächenangleichung zwischen der zweiten und ersten Fläche F2, F1 besteht oder erfolgt ist. Eine Abschaltung der Volumenerweiterung in den Unterkammern wird dann durch eine zweite Abschalteeinheit AF2 erwirkt. Eine entsprechende Signalisierung an den Bediener der Mammographieanlage 1 erfolgt.

## Patentansprüche

1. Vorrichtung mit einer mit mindestens einem Kompressionselement (3.1, 3.2) und mit einer mindestens eine Unterkammer (4.1,...,4.n) aufweisende Positionierungseinheit (4) ausgebildeten Kompressionseinheit (3) zur Komprimierung einer Brust eines Patienten (8), wobei die mindestens eine Unterkammer während einer Fixierungs- bzw. Komprimierungsphase der Brust mit einem Medium patientenindividuell füllbar ist,
wobei weiterhin eine erste Drucksteuerungseinheit (LKS) vorgesehen ist, wobei mit dieser die Komprimierung der Brust des Patienten (8) mit zumindest einem Sensor überwacht wird und die Komprimierung der Brust des Patienten (8) patientenindividuell steuerbar ist, wobei die Abdruckfläche einer zu komprimierenden Brust an das Kompressionselement überwacht und aus den Überwachungsdaten der Anpressdruck der mindestens einen Unterkammer für die Brust des Patienten steuerbar ist, **dadurch gekennzeichnet dass** der zumindest eine Sensor zur optischen Überwachung der zu komprimierenden Brust des Patienten (8) ausgebildet ist, und die Kompressionseinheit (3) zumindest ein mit einer Gaze (3.4) bespannten Rahmen (3.1.1) ausgebildetes Kompressionselement (3.1, 3.2) aufweist, wobei die Gaze (3.4) derart transparent ausgebildet ist,
dass eine Außenkontur (20) der Brust des Patienten (8) sowie eine Ist-Kontaktlinie (21) an der Gaze (3.4) der zu komprimierenden Brust des Patienten (8) optisch erfasst werden kann; und
dass die damit eingeschlossenen Flächen ermittelt werden.

2. Vorrichtung nach Patentanspruch 1,
**dadurch gekennzeichnet,**
**dass** der zumindest eine Sensor eine Aufzeichnungseinheit (7) ist, wobei die zu komprimierende Brust des Patienten (8) mit der Aufzeichnungseinheit (7) während des Komprimierungsvorgangs im Hinblick auf
eine großflächige Anfügung des Gewebes der Brust des Patienten (8) an zumindest ein Kompressionselement (3.1, 3.2) der Kompressionseinheit (3) überwacht wird.

3. Vorrichtung nach einem der vorhergehenden Patentansprüche, **dadurch gekennzeichnet,**
**dass** eine erste Drucksteuerungseinheit (LKS) ein Segmentierungs- und Flächenermittlungsmodul (SFM) mit einer ersten Segmentierungs-/Flächenbestimmeinheit (ESFE) zur Bestimmung einer ersten Fläche (F1) und
einer zweiten Segmentierungs-/Flächenbestimmeinheit (ZSFE) zur Bestimmung einer zweiten Fläche (F2) aufweist.

4. Vorrichtung nach Patentanspruch 3, **dadurch gekennzeichnet,**
**dass** die erste Segmentierungs-/Flächenbestimmeinheit (ESFE) zur Bestimmung der ersten Fläche (F1) die Außenkontur (20) der Brust des Patienten (8) sowie die zum Patenten weisende Kante (3.1.2) des oberen Kompressionselementes (3.1) in den Aufzeichnungen der Aufzeichnungseinheit (7) segmentierbar sind und die durch Außenkontur (20) und die zum Patienten weisende Kante (3.1.2) eines oberen Kompressionselementes (3.1) begrenzte erste Fläche (F1) ermittelbar ist,
**dass** die zweite Segmentierungs-/Flächenbestimmeinheit (ESFE) zur Bestimmung der zweiten Fläche (F2) eine Ist-Kontaktlinie (21) sowie die zum Patenten weisende Kante (3.1.2) des oberen Kompressionselementes (3.1) segmentiert und die durch Ist-Kontaktline (21) und Kante (3.1.2) des oberen Kompressionselementes (3.1) begrenzte zweite Fläche (F2) ermittelbar ist.

5. Vorrichtung nach Patentanspruch 4, **dadurch gekennzeichnet,**
**dass** die erste Drucksteuerungseinheit (LKS) eine zweite Drucksteuerungseinheit (PDE) aufweist, wobei die zweite Drucksteuerungseinheit (PDE) das Volumen in mindestens einem Luftkissen (4.1,...,4.n) der Positionierungseinheit (4) derart verändert, dass die darin eingebettete Brust des Patienten (8) an das zumindest eine Kompressionselement (3.1, 3.2) der Kompressionseinheit (3) gedrückt wird, wobei in mindestens einem selektierten Bereich der Druck auf die in der Kompressionseinheit (3) fixierten Brust derart veränderbar ist, dass ein Ausmaß der zweiten Fläche (F2) einem Ausmaß der ersten Fläche (F1) in zumindest diesem selektierten Bereich entspricht.

6. Vorrichtung nach einem der vorhergehenden Patentansprüche, **dadurch gekennzeichnet,**
**dass** zumindest in ein Kompressionselement (3.1, 3.2) der Kompressionseinheit (3) eine Ultraschalleinheit (6) integriert ist.

7. Vorrichtung nach einem der vorhergehenden Patentansprüche, **dadurch gekennzeichnet**,
das diese in einer Mammographieanlage (1) integriert ist.

8. Verfahren mit einer mit mindestens einem Kompressionselement (3.1, 3.2) und mit einer mindestens eine Unterkammer (4.1,...,4.n) aufweisende Positionierungseinheit (4) ausgebildeten Kompressionseinheit (3) zur Komprimierung einer Brust eines Patienten (8),
wobei die Komprimierung der Brust des Patienten (8) sensor-überwacht patientenindividuell gesteuert wird, wobei die Abdruckfläche einer zu komprimierenden Brust an das Kompressionselement überwacht wird und aus den Überwachungsdaten der Anpressdruck der mindestens einen Unterkammer für die Brust des Patienten gesteuert wird, wobei
der zumindest eine Sensor die zu komprimierende Brust des Patienten (8) optisch überwacht, und die Kompressionseinheit (3) zumindest ein mit einer Gaze (3.4) bespannten Rahmen (3.1.1) ausgebildetes Kompressionselement (3.1, 3.2) aufweist, wobei die Gaze (3.4) derart transparent ausgebildet ist,
dass eine Außenkontur (20) der Brust des Patienten (8) sowie eine Ist-Kontaktlinie (21) an der Gaze (3.4) der zu komprimierenden Brust des Patienten (8) optisch erfasst werden kann und
wobei die damit eingeschlossenen Flächen ermittelt werden.

9. Verfahren nach Patentanspruch 8,
**dadurch gekennzeichnet,**
**dass** nach einer großflächigen Anfügung des Gewebes der Brust des Patienten (8) an zumindest ein Kompressionselement (3.1, 3.2) der Kompressionseinheit (3) der Kompressionsvorgang zur Komprimierung der Brust beendet wird.

10. Verfahren nach einem der vorhergehenden Patentansprüche 8 oder 9,
**dadurch gekennzeichnet, dass**
aufgrund der ermittelten Flächen partiell Flächenunterschiede ermittelt und diese ausgeglichen werden.

11. Verfahren nach einem der vorhergehenden Patentansprüche 8 bis 10,
**dadurch gekennzeichnet, dass** eine erste Drucksteuerungseinheit (LKS) ein Segmentierungs- und Flächenermittlungsmodul (SFM) mit einer ersten Segmentierungs-/ Flächenbestimmeinheit (ESFE) zur Bestimmung einer ersten Fläche (F1) und einer zweiten Segmentierungs-/Flächenbestimmeinheit (ZSFE) zur Bestimmung einer zweiten Fläche (F2) aufweist;
dass das Volumen in mindestens einer Unterkammer (4.1,...,4.n) der Positionierungseinheit (4) derart verändert wird,
dass die darin eingebettete Brust des Patienten (8) an das zumindest eine Kompressionselement (3.1, 3.2) der Kompressionseinheit (3) gedrückt wird, wobei in mindestens einem Bereich der Druck auf die in der Kompressionseinheit (3) fixierte Brust derart veränderbar ist,
dass ein Ausmaß der zweiten Fläche (F2) einem Ausmaß der ersten Fläche (F1) in zumindest diesem Bereich angenähert wird.

## Claims

1. Apparatus having a compression unit (3) for compressing a breast of a patient (8), which compression unit is formed by at least one compression element (3.1, 3.2) and by a positioning unit (4) comprising at least one sub-chamber (4.1, ..., 4.n), wherein the at least one sub-chamber can be filled with a medium in a patient-specific manner during the immobilisation and/or compression phase of the breast, wherein furthermore a first pressure control unit (LKS) is provided, by means of which the compression of the breast of the patient (8) is monitored using at least one sensor, and the compression of the breast of the patient (8) can be controlled in a patient-specific manner, wherein the impression area of a breast to be compressed is monitored, and the contact pressure of the at least one sub-chamber for the breast of the patient can be controlled from the monitoring data, **characterised in that** the at least one sensor is formed to visually monitor the breast of the patient (8) that is to be compressed, and the compression unit (3) comprises at least one compression element (3.1, 3.2) formed by a frame (3.1.1) covered by a gauze (3.4), wherein the gauze (3.4) is designed to be transparent in such a manner that
it is possible to visually capture an outer contour (20) of the breast of the patient (8) and an actual contact-line (21) of the breast of the patient (8) that is to be compressed on the gauze (3.4); and
the areas enclosed thereby are determined.

2. Apparatus according to claim 1,
**characterised in that**
the at least one sensor is a recording unit (7), wherein the breast of the patient (8) that is to be compressed is monitored by the recording unit (7) during the compression process with regard to tissue of the breast of the patient (8) adjoining over a large area at least one compression element (3.1, 3.2) of the compression unit (3).

3. Apparatus according to one of the preceding claims, **characterised in that**
a first pressure control unit (LKS) comprises a segmentation/area-determination module (SFM) having a first segmentation/area-defining unit (ESFE) for defining a first area (F1) and
a second segmentation/area-defining unit (ZSFE) for defining a second area (F2).

4. Apparatus according to claim 3,
**characterised in that**
the first segmentation/area-defining unit (ESFE) for defining the first area (F1) is able to segment, in the recordings from the recording unit (7), the outer contour (20) of the breast of the patient (8) and the edge (3.1.2) of the top compression element (3.1) that faces the patient, and is able to determine the first area (F1), which is bounded by outer contour (20) and the edge (3.1.2) of a top compression element (3.1) that faces the patient,
the second segmentation/area-defining unit (ESFE) for defining the second area (F2) segments an actual contact-line (21) and the edge (3.1.2) of the top compression element (3.1) that faces the patient, and is able to determine the second area (F2), which is bounded by actual contact-line (21) and edge (3.1.2) of the top compression element (3.1).

5. Apparatus according to claim 4,
**characterised in that**
the first pressure control unit (LKS) comprises a second pressure control unit (PDE), wherein the second pressure control unit (PDE) changes the volume in at least one air cushion (4.1,...,4.n) of the positioning unit (4) such that the breast of the patient (8) that is embedded therein is pushed onto the at least one compression element (3.1, 3.2) of the compression unit (3), wherein in at least one selected region the pressure on the breast immobilised in the compression unit (3) can be changed such that a dimension of the second area (F2) matches a dimension of the first area (F1) in at least this selected region.

6. Apparatus according to one of the preceding claims, **characterised in that**
an ultrasound unit (6) is integrated at least in a compression element (3.1, 3.2) of the compression unit (3).

7. Apparatus according to one of the preceding claims, **characterised in that**
this apparatus is integrated in a mammography system (1).

8. Method having a compression unit (3) for compressing a breast of a patient (8), which compression unit is formed by at least one compression element (3.1, 3.2) and by a positioning unit (4) comprising at least one sub-chamber (4.1, ..., 4.n),
wherein the compression of the breast of the patient (8) is controlled under sensor monitoring in a patient-specific manner, wherein the impression area of a breast to be compressed is monitored, and the contact pressure of the at least one sub-chamber for the breast of the patient can be controlled from the monitoring data, wherein the at least one sensor visually monitors the breast of the patient (8) that is to be compressed, and the compression unit (3) comprises at least one compression element (3.1, 3.2) formed by a frame (3.1.1) covered by a gauze (3.4), wherein the gauze (3.4) is designed to be transparent in such a manner that it is possible to visually capture an outer contour (20) of the breast of the patient (8) and an actual contact-line (21) of the breast of the patient (8) that is to be compressed on the gauze (3.4); and
wherein the areas enclosed thereby are determined.

9. Method according to claim 8,
**characterised in that**
the compression process for compressing the breast is terminated once the tissue of the breast of the patient (8) adjoins over a large area at least one compression element (3.1, 3.2) of the compression unit (3).

10. Method according to one of the previous claims 8 or 9, **characterised in that**
on the basis of the determined areas, area differences are determined partially and cancelled out.

11. Method according to one of the previous claims 8 to 10, **characterised in that** a first pressure control unit (LKS) comprises a segmentation/area-determination module (SFM) having a first segmentation/area-defining unit (ESFE) for defining a first area (F1) and a second segmentation/area-defining unit (ZSFE) for defining a second area (F2);
the volume in at least one sub-chamber (4.1, ..., 4.n) of the positioning unit (4) is changed such that
the breast of the patient (8) that is embedded therein is pushed onto the at least one compression element (3.1, 3.2) of the compression unit (3), wherein in at least one region, the pressure on the breast immobilised in the compression unit (3) can be changed such that
a dimension of the second area (F2) converges with a dimension of the first area (F1) in at least this region.

## Revendications

1. Dispositif comprenant une unité (3) de compression pour la compression du sein d'une patiente (8) constituée d'une unité (4) de mise en position comportant au moins un élément (3.1, 3.2) de compression et comportant au moins une sous-chambre (4.1, ..., 4.n), dans lequel la au moins une sous-chambre peut, pendant une phase d'immobilisation et respectivement de compression du sein, être remplie individuellement au patient d'un fluide,
dans lequel en outre
il est prévu une première unité (LKS) de commande de la pression, dans lequel, par celle-ci, on contrôle la compression du sein de la patiente (8) par au moins un capteur,
et la compression du sein de la patiente (8) peut être commandée individuellement à la patiente, dans lequel la surface d'empreinte d'un sein à comprimer sur l'élément de compression est contrôlée et, à partir des données de contrôle, la pression d'application de la au moins une sous-chambre peut être commandée pour le sein de la patiente, **caractérisé en ce que** le au moins un capteur est constitué pour le contrôle optique du sein à comprimer de la patiente (8) et l'unité (3) de compression a au moins un élément (3.1, 3.2) constitué d'un cadre (3.1.1), sur lequel est tendue une gaze (3.4), la gaze (3.4) étant transparente,
de manière à ce qu'un contour (20) extérieur du sein de la patiente (8) ainsi qu'une ligne (21) de contact réel sur la gaze (3.4) du sein à comprimer de la patiente (8) puissent être détectés optiquement ; et
de manière à déterminer les surfaces ainsi enfermées.

2. Dispositif suivant la revendication 1,
**caractérisé**
**en ce que** le au moins un capteur est une unité (7) d'enregistrement, dans lequel on contrôle le sein à comprimer de la patiente (8) par l'unité (7) d'enregistrement pendant l'opération de compression en ce qui concerne un accolement de grande surface du tissu du sein de la patiente (8) à au moins un élément (3.1, 3.2) de compression de l'unité (3) de compression.

3. Dispositif suivant l'une des revendications précédentes, **caractérisé**
**en ce qu'**une première unité (LKS) de commande de la pression comporte un module (SFM) de segmentation et de détermination de surface ayant une première unité (ESFE) de segmentation/détermination de surface pour la détermination d'une première surface (F1) et
une deuxième unité (ZSFE) de segmentation/détermination de surface pour la détermination d'une deuxième surface (F2).

4. Dispositif suivant la revendication 3,
**caractérisé**
**en ce que** la première unité (ESFE) de segmentation/détermination de surface, pour la détermination de la première surface (F1), le contour (20) extérieur du sein de la patiente (8) ainsi que le bord (3.1.2), tourné vers la patiente, de l'élément (3.1) de compression supérieur, peuvent être segmentés dans les enregistrements de l'unité (7) d'enregistrement et la première surface (F1), délimitée par le contour (20) extérieur et le bord (3.1.2), tourné vers la patiente, d'un élément (3.1) de compression supérieur peut être déterminée,
**en ce que** la deuxième unité (ESFE) de segmentation/détermination de surface, pour la détermination de la deuxième surface (F2), segmente une ligne (21) de contact réel ainsi que le bord (3.1.2), tourné vers la patiente, de l'élément (3.1) de compression supérieur et la deuxième surface (F2), délimitée par la ligne (21) de contact réel et le bord (3.1.2) de l'élément (3.1) de compression supérieur, peut être déterminée.

5. Dispositif suivant la revendication 4,
**caractérisé**
**en ce que** la première unité (LKS) de commande de la pression comporte une deuxième unité (PDE) de commande de la pression, dans lequel la deuxième unité (PDE) de commande de la pression modifie le volume, dans au moins un coussin (4.1, ..., 4.n) d'air de l'unité (4) de mise en position, de manière à ce que le sein de la patiente (8), qui y est mis, soit pressé sur le au moins un élément (3.1, 3.2) de compression de l'unité (3) de compression, dans lequel, dans au moins une partie sélectionnée, la pression sur le sein, immobilisé dans l'unité (3) de compression, peut être modifiée, de manière à ce qu'une mesure de la deuxième surface (F2) corresponde à une mesure de la première surface (F1) dans au moins cette partie sélectionnée.

6. Dispositif suivant l'une des revendications précédentes, **caractérisé**
**en ce qu'**une unité (6) à ultrasons est intégrée au moins dans un élément (3.1, 3.2) de compression de l'unité (3) de compression.

7. Dispositif suivant l'une des revendications précédentes, **caractérisé**
**en ce que** celui-ci est intégré dans une installation (1) de mammographie.

8. Procédé par une unité (3) de compression du sein d'une patiente (8) et comprenant une unité (4) de mise en position comportant au moins un élément (3.1, 3.2) de compression et au moins une sous-chambre (4.1, ..., 4n),
dans lequel
on commande la compression du sein de la patiente (8) individuellement à la patiente, en la contrôlant par un capteur, dans lequel on contrôle la surface d'empreinte d'un sein à comprimer sur l'élément de compression et, à partir des données de contrôle, on commande la pression d'application de la au moins une sous-chambre pour le sein de la patiente, dans lequel le au moins un capteur contrôle optiquement le sein à comprimer de la patiente (8) et l'unité (3) de compression a au moins un élément (3.1, 3.2) de compression constitué d'un cadre (3.1.1) sur lequel est tendue une gaze (3.4), dans lequel la gaze (3.4) est constituée de manière transparente,
de manière à ce qu'un contour (20) extérieur du sein de la patiente (8) ainsi qu'une ligne (21) de contact réel sur la gaze (3.4) du sein à comprimer de la patiente (8) puisse être détectée optiquement ;
et
de manière à déterminer les surfaces ainsi enfermées.

9. Procédé suivant la revendication 8,
**caractérisé**
**en ce que**, après un accolement de grande surface du tissu du sein de la patiente (8) à au moins un élément (3.1, 3.2) de compression de l'unité (3) de compression, on met fin à l'opération de compression du sein.

10. Procédé suivant l'une des revendications 8 ou 9 précédentes, **caractérisé**
**en ce que** l'on détermine des différences de surfaces, en partie sur la base des surfaces déterminées et on les compense.

11. Procédé suivant l'une des revendications 8 à 10 précédentes, **caractérisé**
**en ce qu'**une première unité (LKS) de commande de la pression comporte un module (SFM) de segmentation et de détermination de surface ayant une première unité (ESFE) de segmentation/détermination de surface pour la détermination d'une première surface (F1) et une deuxième unité (ZSFE) de segmentation/détermination de surface pour la détermination d'une deuxième surface (F2) ;
**en ce que** l'on modifie le volume, dans au moins un coussin (4.1, ..., 4.n) de l'unité (4) de mise en position, de manière à ce que le sein de la patiente (8), qui y est mis, soit pressé sur le au moins un élément **(3.1,** 3.2) de compression de l'unité (3) de compression, dans lequel, dans au moins une partie sélectionnée, la pression sur le sein, immobilisé dans l'unité (3) de compression, peut être modifiée,
**en ce qu'**une mesure de la deuxième surface (F2) se rapproche d'une mesure de la première surface (F1) dans au moins cette partie sélectionnée.
